# EUROPÄISCHE PATENTANMELDUNG

(11) **EP 1 582 595 A1**
(43) Veröffentlichungstag der Anmeldung: **05.10.2005**
(21) Anmeldenummer: 05006206.6
(22) Anmeldetag: 22.03.2005
(51) Int. Cl.: C12P 7/64

(54) **Enzymatisches Verfahren zur Herstellung von Triglyzeriden, ausgehend von Alkylestern mehrfach ungesättigter Fettsäuren**

(30) Priorität: 31.03.2004 DE 102004015782
(71) Anmelder: Cognis IP Management GmbH, 40589 Düsseldorf (DE)
(72) Erfinder: Schörken, Ulrich, 40591 Düsseldorf (DE); Meyer, Carolin, 40589 Düsseldorf (DE); Both, Sabine, 41470 Neuss (DE); Horlacher, Peter, 89287 Bellenberg (DE)

(57) **Zusammenfassung**

Vorgeschlagen wird ein Verfahren zur enzymkatalysierten Synthese von Triglyceriden mit mehrfach ungesättigten Fettsäuren, bei dem man der Synthese eine Vorhydrolyse vorschaltet, in dem
(a) C₁ bis C₄-Alkylester mehrfach ungesättigter Fettsäuren unter Vakuum und unter kontinuierlicher Dosierung von Wasser komplett oder partiell hydrolysiert oder in mehreren Stufen ohne Vakuum partiell hydrolysiert werden,
(b) nach der Hydrolyse das Wasser über Separation oder Destillation entfernt wird, und nachfolgend
(c) die Synthese der mehrfach ungesättigten Fettsäuren mit Glycerin unter Vakuum in ihre Triglyceride in Gegenwart eines immobilisierten Enzyms unter wasserarmen Bedingungen durchgeführt wird,
(d) die immobilisierten Enzyme vom Triglycerid über Separation oder Filtration abtrennt werden und
(e) die restlichen Fettsäuren und/oder mögliche Reste an C₁ bis C₄-Alkylestern destillativ oder über Raffination vom Triglycerid entfernt werden.

Vorzugsweise wird die Vorhydrolyse (Schritte a und b) und die nachfolgende Synthese (Schritte c bis e) als Eintopfverfahren im selben Reaktor und in Gegenwart des gleichen immobilisierten Enzyms durchgeführt. Vorteil dieses Verfahrens ist eine bessere Stabilisierung des Enzyms.

## Beschreibung

### Gebiet der Erfindung

Die Erfindung befindet sich auf dem Gebiet der Fettsäureester und betrifft ein neues Verfahren zur enzymatischen Synthese von Triglyceriden, die mehrfach ungesättigte Fettsäuren enthalten, das sich aus einer Vorhydrolyse von Alkylestern mehrfach ungesättigter Fettsäuren unter wässriger Umgebung und einer Umsetzung der dabei freigesetzten Fettsäuren zu Triglyceriden unter wasserarmen Bedingungen zusammensetzt.

### Stand der Technik

Die Herstellung von Estern mehrfach ungesättigter Fettsäuren kann auf chemischem Wege wie auch auf enzymatischem Wege erfolgen. Chemische Synthesen haben den Nachteil, dass in der Regel sehr hohe Temperaturen eingesetzt werden müssen und große Mengen an basischen Katalysatoren benötigt werden, wodurch in höherem Maße Nebenprodukte und unerwünschte Isomerisierungen auftreten. Enzymatisch katalysierte Umsetzungen mit Lipasen finden in der Regel unter milderen Bedingungen statt und ergeben Endprodukte hoher Reinheit.
In der Europäischen Patentanmeldung **EP 1 322 776** A1 wird eine lipase katalysierte Methode zur Herstellung von Triglyceriden mehrfach ungesättigter konjugierter Fettsäuren ausgehend vom Alkylester der ungesättigten Fettsäuren und Glycerin beschrieben, die den entstehenden Alkohol unter vermindertem Druck aus der Reaktion entfernt. Auch aus der Internationalen Patentanmeldung **WO 9116443 A1** ist die Veresterung von Glycerol und freien mehrfach ungesättigten Fettsäuren oder deren Alkylestern zu den entsprechenden Triglyceriden durch Entfernung des Reaktionswassers bzw. des entstehenden Alkohols unter vermindertem Druck beschrieben.
Enzymatische Synthesen haben jedoch häufig den Nachteil, dass die Umsetzungen relativ langsam ablaufen. Die Stabilität der meisten Enzyme unter sehr wasserarmen Bedingungen wie beispielsweise bei der direkten Umesterung von Alkylestern zu Glyceriden ist gering. Setzt man die freie Säure ein, was die eigentliche Triglyceridsynthese beschleunigt, so müssen Ester ungesättigter Fettsäuren dennoch vorher hydrolysiert werden.

Die Aufgabe der vorliegenden Erfindung hat daher darin bestanden, enzymatische Verfahren zur Herstellung von Triglyceriden mit mehrfach ungesättigten Fettsäuren in ihrer Rentabilität zu verbessern.

### Beschreibung der Erfindung

Gegenstand der Erfindung ist ein Verfahren zur enzymkatalysierten Synthese von Triglyceriden mit mehrfach ungesättigten Fettsäuren, bei dem man der Synthese eine Vorhydrolyse vorschaltet, in dem
(a) C₁ bis C₄-Alkylester mehrfach ungesättigter Fettsäuren unter Vakuum und unter kontinuierlicher Dosierung von Wasser komplett oder partiell hydrolysiert oder in mehreren Stufen ohne Vakuum partiell hydrolysiert werden,
(b) nach der Hydrolyse das Wasser über Separation oder Destillation entfernt wird, und nachfolgend
(c) die Synthese der mehrfach ungesättigten Fettsäuren mit Glycerin unter Vakuum in ihre Triglyceride in Gegenwart eines Enzyms unter wasserarmen Bedingungen durchgeführt wird,
(d) die Enzyme vom Triglycerid über Separation oder Filtration abtrennt werden und
(e) die restlichen Fettsäuren und/oder mögliche Reste an C₁ bis C₄-Alkylestern destillativ oder über Raffination vom Triglycerid entfernt werden.
Vorzugsweise wird die Vorhydrolyse (Schritte a und b) und die nachfolgende Synthese (Schritte c bis e) als Eintopfverfahren im selben Reaktor durchgeführt und insbesondere in Gegenwart des gleichen Enzyms. Vorteil dieses Verfahrens ist eine bessere Stabilisierung des Enzyms zur Triglyceridsynthese.
Es wurde gefunden, dass die Stabilität und damit die Wiederverwendbarkeit des Enzyms entscheidend verbessert wird und somit die Kosten des Verfahrens gesenkt werden können, da das Enzym in der Regel der kostenintensivste Faktor eines solchen Syntheseverfahrens ist.

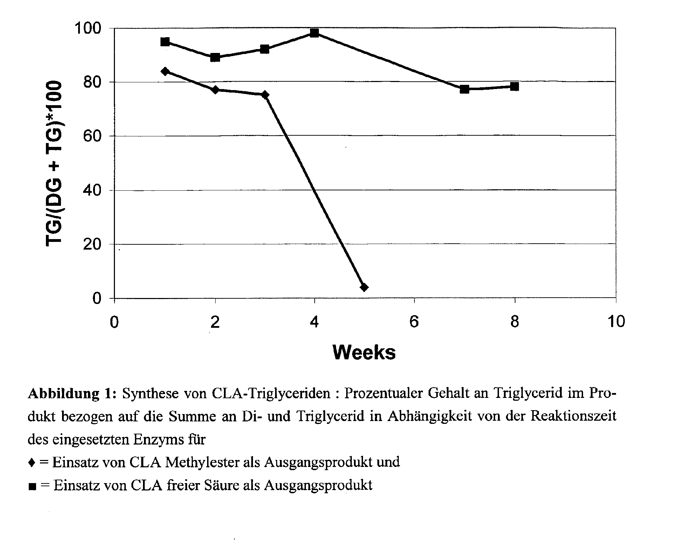

Fig. 1 beschreibt die Umsetzung von CLA Methylester (◆) oder freier Säure CLA (■) mit Glycerin zu CLA-Triglyceriden in Gegenwart der immobilisierten Lipase Novozym 435 (Lipase von Novozymes, Dänemark). Es zeigte sich, dass bei Einsatz der freien Säure eine geringere Abnahme der Enzymaktivität zu beobachten ist. So konnte nach 5 Wochen das Novozym 435 im Ansatz mit CLA-Methylester nicht mehr filtriert werden. Die Verwendung von freier Säure setzt jedoch eine Esterspaltung der vorwiegend in Form von Estern vorliegenden CLA voraus. Würde man wie im Stand der Technik bekannt, die Ester direkt mit dem Enzym in Form einer Umesterung umsetzen, würde das Enzym erhebliche Stabilitätseinbußen erfahren (siehe Fig. 1).
Das vorliegende Verfahren setzt sich daher aus zwei Schritten zusammen, in denen eine Vorhydrolyse der eigentlichen Synthese vorangestellt wird.
Vor der Veresterung wird vorzugsweise im gleichen Reaktionsgefäss in wasserreichem Medium der CLA-Alkylester in Alkohol und die freie Säure gespalten. Unter "wasserreich" wird eine Wassermenge von maximal 50 %, vorzugsweise maximal 25 % und mindestens 1 % vorzugsweise mindestens 5 % Wasser bezogen auf den Gesamtansatz verstanden. Über kontinuierliche Zudosierung von Wasser wird auch unter Vakuum ein Gehalt von mindestens 1 % vorzugsweise mindestens 5 % Wasser im Ansatz bezogen auf den Gesamtansatz eingestellt. Die hydrolytische Spaltung kann auf hinreichend bekanntem chemischem Wege durchgeführt werden. Vorzugsweise wir jedoch auch die Vorhydrolyse in Gegenwart von Enzymen durchgeführt und insbesondere in Gegenwart des gleichen Enzyms das in der nachfolgenden Synthese verwendet wird.
Die Synthese der Triglyceride wird unter wasserarmen Bedingungen gefahren. Darunter ist zu verstehen, dass die Synthese unter Vakuum ohne die Zudosierung von Wasser oder Wasserdampf durchgeführt wird. Geringe Mengen an Wasser entstehen in der Synthese kontinuierlich durch die Bildung der Ester aus Glycerin und Fettsäure. Dieses Reaktionswasser schützt die Lipase vor kompletter Dehydratisierung.
Dagegen wird bei Einsatz der Ester als Substrate kein Reaktionswasser gebildet, so dass es unter Vakuum schneller zu einer Dehydratisierung der Lipase und damit zu einer Deaktivierung kommt.
Verglichen mit der chemischen Synthese von Triglyceriden mehrfach ungesättigter Fettsäuren kann bei wesentlich geringeren Temperaturen gearbeitet werden, was zu einer Verminderung unerwünschter Nebenprodukte wie z.B. unerwünschter Isomere führt. Normalerweise ist die Reaktionsgeschwindigkeit dieser enzymatischen Prozesses sehr niedrig. Das erfindungsgemäße Verfahren führt jedoch zu einer Stabilisierung der eingesetzten Enzyme, die somit Wiederverwendung finden und das enzymatische Verfahren zu einem rentablen Prozess machen.

Das Verfahren ist anwendbar für C₁ bis C₄-Alkylester vorzugsweise Methyl- und/oder Ethylester auswählt aus der Gruppe, die gebildet wird von natürlich vorkommenden mehrfach ungesättigten und mehrfach konjugiert ungesättigten Fettsäuren sowie konjugierten Linol- und Linolensäuren. Vorzugsweise werden Ester der Docosahexaensäure, Eicosapentaensäure, Arachidonsäure, gamma-Linolensäure und konjugierte Linolsäure, dabei besonders bevorzugt die c9,t11 und t10,c12 Isomere der konjugierten Linolsäure (CLA) eingesetzt. Der gewählte Konzentrationsbereich der eingesetzten Rohstoffe liegt bei 3 bis 6 mol Ester auf 1 mol Glycerin, bevorzugt werden 3,2 bis 4,0 mol Ester pro 1 mol Glycerin eingesetzt, um eine optimale Umsetzungsgeschwindigkeit zu erreichen.

Typische Beispiele für geeignete Enzyme, die jedoch nicht einschränkend sein sollen, sind Lipasen und/oder Esterasen von Mikroorganismen die ausgewählt sind aus der Gruppe, die gebildet wird von Alcaligenes, Aspergillus, Candida, Chromobacterium, Rhizomucor, Penicilium, Pseudomonas, Rhizopus, Thermomyces, Geotrichum, Mucor, Burkholderia sowie deren Gemischen. Bevorzugt, weil besonders aktiv, sind Lipasen und Esterasen aus den Organismen vom Typ Alcaligenes, Candida, Chromobacterium, Penicilium, Pseudomonas, Rhizopus, Rhizomucor und Thermomyces, besonders bevorzugt sind darunter Candida und Rhizopus, speziell Candida antarctica B. Es werden bevorzugt Lipasen immobilisiert auf Trägermaterial eingesetzt, insbesondere 3 bis 12 Gew. % Immobilisat bezogen auf den Fettanteil.
Der für die Reaktion geeignete Temperaturbereich richtet sich nach dem Aktivitätsoptimum der Enzyme. Für die bevorzugt ausgewählten Lipasen haben sich 40 bis 90° C als besonders geeignet erwiesen, speziell bevorzugt wird der Bereich von 55 bis 80°C. Dabei sollte ein Vakuum von mindestens 200 mbar, bevorzugt 1 bis 100 mbar und besonders bevorzugt 20 bis 60 mbar angelegt werden. Die bevorzugten Versuchsparameter ergeben sich aus der zu erreichenden Beschleunigung der Reaktionsgeschwindigkeit.

Nach erfolgter Umsetzung werden die immobilisierten Enzyme durch Separation oder Filtration abgetrennt und die nicht umgesetzten Fettsäuren oder deren Alkylester durch Raffination oder Destillation, vorzugsweise Kurzwegdestillation entfernt.

Zusätzlich zum Einsatz der Vorhydrolyse kann das Verfahren weiterhin optimiert werden, dadurch dass man den Schritt c beschleunigt durch
(i) Zugabe eines Additivs aus der Gruppe der schwach basischen Salze, Komplexierungsmittel und basischen Ionenaustauscher und/oder
(ii) Zugabe eines Schleppmittels in Form eines Lösungsmitels oder eines Gases und/oder
(iii) Zugabe Glycerin bindender Adsorber und/oder
(iv) thermische Behandlung des Partialglycerid-Zwischenproduktes.

### Beispiele

### Beispiel 1

### Vergleich der Stabilitäten von Novozym 435 in CLA Methylester und CLA freier Säure unter Betriebsbedingungen

In einen Kolben werden Glycerin (4 g) und CLA-Fettsäure (44 g) in einem Molverhältnis von 1 : 3,6 und in einem zweiten Kolben werden Glycerin (5,5 g) und CLA Methylester (66 g) in einem Molverhältnis von 1 : 3,7 eingewogen. Nach Zugabe von Novozym® 435 (Lipase von Novozymes, Dänemark) (4 g in Ansatz 1 und 3 g in Ansatz 2) wird bei einer Temperatur von 60 °C unter Rühren mit einem Magnetrührfisch ein Vakuum von 20 mbar angelegt. Nach 48 h wird eine Probe der Ölphase entnommen und gaschromatographisch auf den Anteil an Glyceriden untersucht. Novozym 435 wird über Filtration vom gebildeten CLA-Triglycerid abgetrennt, in den Kolben zurückgeführt und eine neue Reaktion unter gleichen Reaktionsbedingungen wird gestartet. In dieser Weise werden Reaktionen über einen Zeitraum von 8 Wochen durchgeführt. Die Reaktion ausgehend vom CLA Methylester wird unter kontinuierlicher Stickstoffbegasung durchgeführt.

Ergebnis (siehe Abbildung 1):
◆ = Einsatz von CLA Methylester
■ = Einsatz von CLA freier Säure
Es wird der prozentuale Gehalt an Triglycerid im Produkt bezogen auf die Summe an Di- und Triglycerid bestimmt. Es zeigt sich deutlich, dass bei Einsatz des CLA-Methylesters zur Synthese von CLA-Triglyceriden bereits nach 5 Wochen die Kapazität des eingesetzten Enzyms erschöpft ist und das zur reinen Veresterung eingesetzte Enzym nach 8 Wochen noch über 80% seiner ursprünglichen Kapazität aufweist. Bei Einsatz von CLA freier Säure ist nur eine geringere Abnahme der Enzymaktivität zu beobachten, was sowohl auf Deaktivierung des Enzyms wie auch stärkeren Abrieb des Trägermaterials zurückzuführen ist.

### Beispiel 2

### Selektion geeigneter Lipasen für die Vorhydrolyse von CLA Estern

15 Ansätze mit jeweils 4 g konjugiertem Linolsäureethylester und 6 g Wasser in einem verschließbaren Reaktionsgefäß wurden bei Raumtemperatur parallel auf einer Multirührplatte gerührt. Zu den Ansätzen werden jeweils 40 mg kommerziell erhältliche Lipasen bzw. Esterasen zudosiert. Nach 2 h und 22 h Reaktionszeit werden jeweils Proben genommen. Die organische Phase enthaltend Fettsäureethylester und enzymatisch hydrolysierte Fettsäure wurden separiert und analysiert. Der Umsatz wurde über die Säurezahl bestimmt. Die Ergebnisse sind in Tabelle 1 zusammengefasst.

**Tabelle 1:**

| Auswahl geeigneter Lipasen und Esterasen zum Verfahren gemäß Anspruch 1 | | | | | | |
|---|---|---|---|---|---|---|
| **Enzym** | **Mikroorganismus** | **Hersteller** | **Säurezahl** | | **Umsatz** | |
| | | | 2h | 22h | 2 h | 22 h |
| Chirazym L-10 | *Alcaligenes sp.* | Roche | 21 | 41 | 11,5 | 20,5 |
| Lipase A | *Aspergillus niger* | Amano | 6 | 16 | 3 | 8 |
| Novozym 868 | *Candida antarctica A* | Novozymes | 5 | 6 | 2,5 | 3 |
| Novozym 525 | *Candida antarctica B* | Novozymes | 52 | 62 | 26 | 30 |
| Lipomod 34 | *Candida cylindracea* | Biocatalysts | 45 | 61 | 22,5 | 30 |
| Lipase LP | *Chromobacterium viscosum* | Asahi Kasei | 45 | 60 | 22,5 | 30 |
| Novozym 388 | *Rhizomucor miehei* | Novozymes | 8 | 11 | 4 | 5,5 |
| Lipase G | *Penicilium camenberti* | Amano | 15 | 38 | 7,5 | 19 |
| Lipase R | *Penicilium roqueforti* | Amano | 6 | 6 | 3 | 3 |
| Lipase L115P | *Porcine pancreas* | Biocatalysts | 6 | 6 | 3 | 3 |
| Lipase PS | *Pseudomonas cepacia* | Amano | 46 | 57 | 23 | 28,5 |
| Lipase AK | *Pseudomonas fluorescens* | Amano | 26 | 53 | 13 | 26,5 |
| Lipomod 36 P | *Rhizopus javanicus* | Biocatalysts | 21 | 38 | 11,5 | 19 |
| Lipase F-AP 15 | *Rhizopus oryzae* | Amano | 12 | 18 | 6 | 9 |
| Lipolase T1 100 | *Thermomyces lanugenosus* | Novozymes | 38 | 53 | 19 | 26,5 |

Alle getesteten Lipasen und Esterasen erwiesen sich in der Hydrolyse der Fettsäureester aktiv. Bevorzugt sind jedoch Mikroorganismen vom Typ Alcaligenes, Candida, Chromobacterium, Penicilium, Pseudomonas, Rhizopus und Thermomyces. Die Reaktion ohne Entfernung von Ethanol reagierte unter obigen Bedingungen bis zu einem Gleichgewicht von etwa 30 Gew.-% freier Fettsäure.

### Beispiel 3

### Hydrolyse von kurzkettigen konjugierten Linolsäuremethylestern unter kontinuierlichem Abstrippen von Wasser und Methanol.

In einen beheizbaren Kolben wurden 100 g konjugierter Linolsäuremethylester, 10 g Wasser und 5 g immobilisierte *Candida antarctica* B Lipase vorgelegt. Die Reaktion wurde mit aufgesetzter Destillationsbrücke bei einem verminderten Druck von 60 mbar und einer Temperatur von 60 °C durchgeführt. Wasser wurde kontinuierlich mit einer Flussrate von 0,25 ml/min (Beispiel 3A) und 0,5 ml/min (Beispiel 3B) in den Kolben gepumpt. Zudosiertes Wasser wurde schnell abdestilliert, so dass der Wassergehalt im Reaktor während der gesamten Reaktionsdauer gering (< 20 %) war. Die Umsetzung der Reaktion wurde über Bestimmung der Säurezahl verfolgt. Die Reaktionen wurden bei Teilumsatz nach 24 h abgebrochen und das immobilisierte Enzym vom Reaktionsgemisch abfiltriert. Dann wurde die organische Phase von der wässrigen Phase über Separation getrennt. Eine Säurezahl von 200 entsprach 100 % Umsatz. Die Ergebnisse sind in Tabelle 4 zusammengefasst:

**Tabelle 4:**

| Umsatz unter Abstrippen von Wasser und Methanol nach unterschiedlichen Reaktionszeiten | | | | |
|---|---|---|---|---|
| **Reaktionszeit [h]** | **Saürezahl Beispiel 3A** | **Umsatz [%] Beispiel 3A** | **Säurezahl Beispiel 3B** | **Umsatz [%] Beispiel 3B** |
| 0 | 0 | 0 | 0 | 0 |
| 2 | 51,5 | 25,7 | 62,3 | 31,1 |
| 4 | 71,2 | 35,6 | 84,4 | 42,4 |
| 6 | 87,0 | 43,5 | 100,2 | 50,1 |
| 8 | 100,0 | 50,0 | 112,2 | 56,1 |
| 24 | 153,0 | 76,5 | 167,1 | 83,5 |

Innerhalb von 24 h wurden je nach Menge des eindosierten Wasser Spaltgrade von 76,5 % bzw. 83,5 % erhalten. Die Destillatmenge in Beispiel 3A betrug nach 24 h 315 g und die destillatmenge in Beispiel 3B 584 g.

### Beispiel 4

### Hydrolyse von kurzkettigen konjugierten Linolsäuremethylestern durch mehrstufige Hydrolyse

In geschlossenen Gefässen wurden 3 Ansätze mit jeweils 20 g konjugiertem Linolsäuremethylester auf Basis von Sonnenblumenöl und 40 g Wasser eingewogen. Anschliessend wurden jeweils 1 g immobilisierte Lipase zugegeben und die Gemische wurden 5 h bei Raumtemperatur auf einer Magnetrührplatte gerührt. Danach wurden die Enzymimmobilisate abfiltriert und die organische Phase wurde von der wässrigen Phase über Separation getrennt. Zur organischen Phase wurden erneut 40 g Wasser gegeben und die abfiltrierten Enzymimmobilisate wurden erneut zur Reaktionslösung gegeben. Nach Reaktion über Nacht bei Raumtemperatur wurden die Enzymimmobilisate erneut abfiltriert und die organische Phase wurde von der wässrigen Phase über Separation getrennt. Zur organischen Phase wurden 40 g Wasser gegeben und die abfiltrierten Enzymimmobilisate wurden erneut zur Reaktionslösung gegeben. Nach weiteren 5 h Reaktion bei Raumtemperatur wurde die Reaktion abgebrochen.
Folgende Enzymimmobilisate wurden eingesetzt:
5A) 1 g Novozym 435
5B) 1 g immobilisierte Candida antarctica B Lipase
5C) 1g immobilisierte Thermomyces lanugenosus Lipase
Die Umsetzung der Reaktion in den einzelnen Stufen wurde über Bestimmung der Säurezahl verfolgt. Eine Säurezahl von 200 entsprach dabei 100 % Umsatz. Die Ergebnisse sind in Tabelle 6 zusammengefasst:

**Tabelle 6:**

| Mehrstufige Hydrolyse von kurzkettigen konjugierten Linolsäuremethylestern | | | | | | |
|---|---|---|---|---|---|---|
| **Reaktions- zeit [h]** | **Säurezahl Beispiel 5A** | **Umsatz [%] Beispiel 5A** | **Säurezahl Beispiel 5B** | **Umsatz [%] Beispiel 5B** | **Säurezahl Beispiel 5C** | **Umsatz [%] Beispiel 5C** |
| 0 | 0 | 0 | 0 | 0 | 0 | 0 |
| Stufe 1, nach 5 h | 100,4 | 50,2 | 81,8 | 40,9 | 74,5 | 37,3 |
| Stufe 2, nach 16 h | 142 | 71 | 127 | 63,5 | 117,2 | 58,6 |
| Stufe 3 Nach 5 h | 165,5 | 82,8 | 154,9 | 77,5 | 152,4 | 76,2 |

### Beispiel 5

### Partialhydrolyse von CLA Methyl- und Ethylester sowie Umsetzung der Hydrolysate mit Glycerin zum CLA Triglycerid

In 2 Kolben werden 300 g Wasser und 900 g CLA-Methylester (Ansatz 1) bzw. 940 g CLA-Ethylester eingewogen. Zu Ansatz 1 werden 0,22 % Natriumcarbonat gegeben. Die Hydrolyse wird über Zugabe von 100 g immobilisierter Candida B Lipase (Ansatz 1) bzw. 80 g immobilisierter Candida B Lipase (Ansatz 1) gestartet. Die Partialhydrolyse wird bei einer Temperatur von 45 °C (Innentemperatur) und einem Vakuum von 60 mbar bei einer Rührerdrehzahl von 300 rpm durchgeführt. Während der Hydrolyse wird kontinuierlich Wasser mit einer Flussrate von 3 ml/min dosiert. Es werden jeweils Proben der Ölphase entnommen und über Säurezahlbestimmung wird der Grad der Hydrolyse bestimmt. Nach 7 h wird die Hydrolyse beendet. Zu den Ansätzen werden jeweils 80 g Glycerin zugegeben und die Glyceridsynthese wird bei einer Temperatur von 55 °C (Innentemperatur) unter Vakuum (60 mbar in Ansatz 1 und 20 mbar in Ansatz 2) durchgeführt. Es werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tabelle 7:**

| Verlauf der Partialhydrolyse von CLA Methyl- und Ethylester und Umsetzung der Hydrolysate mit Glycerin zum CLA Triglycerid | | |
|---|---|---|
| Reaktionszeit | Ansatz 1 | Ansatz 2 |
| 3 h | 60 % Hydrolysegrad | 67 % Hydrolysegrad |
| 6 h | | 84 % Hydrolysegrad |
| 7 h | 91 % Hydrolysegrad | |
| 24 h | 55 % Triglycerid | 22 % Triglycerid |
| 31 h | 61 % Triglycerid | 40 % Triglycerid |
| 48 h | 83 % Triglycerid | 75 % Triglycerid |

Über die kombinierte Hydrolyse von CLA Estern und der Glyceridsynthese ausgehend von den Partialhydrolysaten werden hohe CLA Triglyceridausbeuten erhalten. Bereits nach wenigen Stunden wird ein hoher Hydrolysegrad erreicht. Der Zusatz von Natriumcarbonat (Ansatz 1) führt auch im kombinierten Verfahren zu einer erhöhten Geschwindigkeit der Triglyceridbildung.

## Patentansprüche

1. Verfahren zur enzymkatalysierten Synthese von Triglyceriden mit mehrfach ungesättigten Fettsäuren, bei dem man der Synthese eine Vorhydrolyse vorschaltet, in dem
(a) C₁ bis C₄-Alkylester mehrfach ungesättigter Fettsäuren unter Vakuum und unter kontinuierlicher Dosierung von Wasser komplett oder partiell hydrolysiert oder in mehreren Stufen ohne Vakuum partiell hydrolysiert werden,
(b) nach der Hydrolyse das Wasser über Separation oder Destillation entfernt wird, und nachfolgend
(c)die Synthese der mehrfach ungesättigten Fettsäuren mit Glycerin unter Vakuum in ihre Triglyceride in Gegenwart eines Enzyms unter wasserarmen Bedingungen durchgeführt wird,
(d) die Enzyme vom Triglycerid über Separation oder Filtration abtrennt werden und
(e)die restlichen Fettsäuren und/oder mögliche Reste an C₁ bis C₄-Alkylestern destillativ oder über Raffination vom Triglycerid entfernt werden.

2. Verfahren nach Anspruch 1, **dadurch gekennzeichnet, dass** man die Vorhydrolyse (Stufen a und b) und die nachfolgende Synthese (c bis e) als Eintopfverfahren im selben Reaktor durchführt.

3. Verfahren nach Anspruch 1 und/oder Anspruch 2, **dadurch gekennzeichnet, dass** man die Vorhydrolyse (Schritte a und b) und die nachfolgende Synthese (c bis e) in Gegenwart des gleichen Enzyms durchführt.

4. Verfahren nach mindestens einem der Ansprüche 1 bis 3, **dadurch gekennzeichnet, dass** man die mehrfach ungesättigten Fettsäuren und/oder deren Alkylester auswählt aus der Gruppe, die gebildet wird von Docosahexaensäure, Eicosapentaensäure, Linolsäure und konjugierter Linolsäure.

5. Verfahren nach mindestens einem der Ansprüche 1 bis 4, **dadurch gekennzeichnet, dass** man als Enzyme Lipasen, Phospholipasen oder Esterasen auswählt, die trägergebunden zum Einsatz kommen.

6. Verfahren nach mindestens einem der Ansprüche 1 bis 5, **dadurch gekennzeichnet, dass** man die Vorhydrolyse unter kontinuierlicher Dosierung von Wasser bei einem Vakuum von mindestens 200 mbar durchführt.

7. Verfahren nach mindestens einem der Ansprüche 1 bis 5, **dadurch gekennzeichnet, dass** man die Vorhydrolyse unter kontinuierlicher Dosierung von Wasser in mehreren Stufen ohne anlegen von Vakuum durchführt.

8. Verfahren nach mindestens einem der Ansprüche 1 bis 7, **dadurch gekennzeichnet, dass** man den Schritt a bei mindestens 1 % Wasser und maximal 50 % Wasser im Reaktionsansatz bezogen auf den Gesamtansatz durchführt.

9. Verfahren nach mindestens einem der Ansprüche 1 bis 8, **dadurch gekennzeichnet, dass** man den Schritt c bei einem Vakuum von mindestens 200 mbar ohne Zudosierung von Wasser durchführt.

10. Verfahren nach mindestens einem der Ansprüche 1 bis 9, **dadurch gekennzeichnet, dass** man den Schritt c beschleunigt durch
(i) Zugabe eines Additivs aus der Gruppe der schwach basischen Salze, Komplexierungsmittel und basischen Ionenaustauscher und/oder
(ii) Zugabe eines Schleppmittels in Form eines Lösungsmitels oder eines Gases und/oder
(iii) Zugabe Glycerin bindender Adsorber und/oder
(iv) thermische Behandlung des Partialglycerid-Zwischenproduktes
